(19) Europäisches Patentamt
European Patent Office
Office européen des brevets

(11) Publication number: 0 082 701 B1

(12) **EUROPEAN PATENT SPECIFICATION**

(45) Date of publication of patent specification: **16.07.86**

(51) Int. Cl.[4]: **C 07 C 15/04**, C 07 C 15/06, C 07 C 15/08, C 07 C 1/20

(21) Application number: **82306780.6**

(22) Date of filing: **20.12.82**

(54) **A process for producing a mixture of hydrocarbons rich in benzene, toluene, and xylene.**

(30) Priority: **23.12.81 US 333844**

(43) Date of publication of application:
**29.06.83 Bulletin 83/26**

(45) Publication of the grant of the patent:
**16.07.86 Bulletin 86/29**

(84) Designated Contracting States:
**BE DE FR GB IT NL**

(56) References cited:
**DE-A-3 113 838**
**GB-A-1 446 522**
**US-A-4 076 761**

(73) Proprietor: **MOBIL OIL CORPORATION**
**150 East 42nd Street**
**New York New York 10017 (US)**

(72) Inventor: **Chu, Yung-Feng**
**121 Randle Drive**
**Cherry Hill New Jersey 08034 (US)**
Inventor: **Chester, Arthur Warren**
**517 Country Club Drive**
**Cherry Hill New Jersey 08003 (US)**

(74) Representative: **West, Alan Harry et al**
**Mobil Court 3 Clements Inn**
**London WC2A 2EB (GB)**

EP 0 082 701 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for producing a mixture of hydrocarbons rich in benzene, toluene, and xylene.

Benzene, toluene, and xylenes (BTX) are basic building blocks of the petrochemical industry. The source of these compounds primarily is the refining of petroleum. As petroleum supplies dwindle so does the supply of benzene, toluene and xylene. Alternative sources must be developed for these compounds. We have discovered a process for making benzene, toluene and xylene which utilizes materials which can be indirectly derived from a readily available source, synthesis gas.

The conversion of synthesis gas directly to dimethyl ether has been described in British Patent 278,353 (1926) to F. R. Bechowsky and U.S. Patent 4,098,809 to G. Pagani (1978). The conversion of synthesis gas to oxygenated organic compounds containing mixtures of alcohols, ethers and other compounds is disclosed in a number of patents. U.S. Patent 4,237,063 to Bell et al discloses the conversion of synthesis gas to oxygenated hydrocarbons using metal cyanide complexes. U.S. Patent 4,011,275 discloses the conversion of synthesis gas to methanol and dimethyl ether by passing the mixture over a zinc-chromium acid or copper-zinc-alumina acid catalyst. U.S. Patent 4,076,761 discloses a process for making hydrocarbons wherein coal is converted to synthesis gas, the synthesis gas is converted to oxygen-substituted hydrocarbons comprising methanol and the oxygen-substituted hydrocarbons are converted to liquid hydrocarbon fuels by contacting with a catalytically active alumino silicate zeolite having a silica to alumina ratio of at least 12 and a constraint index of 1 to 12, preferably H-ZSM-5.

Processes for the conversion of coal and other hydrocarbons to a gaseous mixture consisting essentially of hydrogen and carbon monoxide, or of hydrogen and carbon dioxide, or of hydrogen and carbon monoxide and carbon dioxide, are well known. Such a gaseous mixture hereinafter will be referred to simply as synthesis gas.

Although various processes may be employed for the gasification, those of major interest for depend either on the partial combustion of the fuel with oxygen, or on the high temperature reaction of the fuel with steam, or on a combination of these two reactions. In one known variant, for example, coal may be completely gasified by first coking, and then subjecting the coke to a cyclic blue water gas process in which the coke bed is alternately blasted with air to increase the bed temperature and then reacted with steam to produce the synthesis gas. The gases generated by the coking step may be used as fuel or steam-reformed to additional synthesis gas. In another process coal or coke may be reacted with highly superheated steam or with oxygen and steam to produce synthesis gas. Regardless of the process variants chosen, oxygen rather than air is often used in the chemical step in which the fuel is converted to synthesis gas since the use of air would result in a gas that contains excessive amounts of inert nitrogen.

A summary of the art of gas manufacture, including synthesis gas, from solid and liquid fuels, is given in Encyclopedia of Chemical Technology, Edited by Kirk-Othmer, Second Edition, Volume 10, pages 353—433, (1966), Interscience Publishers, New York, N.Y.

It is known that raw synthesis gas contains one or more of the following impurities; sulfur compounds, nitrogen compounds, particulate matter and condensibles. Methods of removing these contaminants are well known, and are described in the above reference and elsewhere. Particular attention is called to the sulfur compounds. It is desirable to reduce this contaminant below a prescribed level for ecological purposes.

Purified synthesis gas ordinarily contains a volume ratio of hydrogen to carbon monoxide plus carbon dioxide of from as little as 0.10 to as much as 1.1, depending on the particular fuel and process used; in most instances, the composition has a volume ratio from 0.30 to 0.65. It is well known that this ratio may be increased by the catalytic carbon-monoxide shift reaction described by the equation:

$$CO+H_2O \rightleftharpoons CO_2+H_2$$

with subsequent removal of at least part of the produced $CO_2$ to bring said volume ratio into a desired high range. The catalytic carbon monoxide shift reaction is commonly conducted with a chromia promoted iron oxide catalyst at a flow rate of 300—1000 standard cubic feet of gas per cubic foot of catalyst bed per hour, and at sufficiently elevated temperature to allow quasi-equilibration, which is usually 371°C (700°F).

Synthesis gas will undergo conversion to form reduction products of carbon monoxide, such as alcohols, at from 149°C (300°F) to 454°C (850°F), under from 1 to 1000 atmospheres ($1\times10^2$ to $1\times10^5$ kPa) pressure, over a fairly wide variety of catalysts. The types of catalyst that induce conversion include ZnO, Fe, Co, Ni, Ru, $ThO_2$, Rh and Os.

Catalysts based on ZnO are particularly suited for the production of methanol and dimethyl ether. Catalysts based on Fe, Co, and Ni, and especially Fe, are particularly suited for the production of oxygenated and hydrocarbon products that have at least one carbon-to-carbon bond in their structure. With the exception of ruthenium, all practical synthesis catalysts contain chemical and structural promoters. These promoters include copper, chromia, alumina and alkali. Alkali is of particular importance with iron catalysts, since it greatly enhances the conversion efficiency of the iron catalyst. Supports such as kieselguhr sometimes act beneficially.

The catalyzed reduction of carbon monoxide or carbon dioxide by hydrogen produces various

oxygenated and hydrocarbon products, depending on the particular catalyst and reaction conditions chosen. The products that are formed include methanol, dimethyl ether, acetone, acetic acid, normal propyl alcohol, higher alcohols, methane, gaseous, liquid, and solid olefins and paraffins. It should be noted that this spectrum of products consists of aliphatic compounds; aromatic hydrocarbons either are totally absent or are formed in minor quantities.

In general, when operating at the lower end of the temperature range, i.e. from 300° to 500°F (149° to 260°C), in the reduction of carbon monoxide, and with pressures greater than 20 atmospheres ($2\times10^3$ kPa), thermodynamic considerations suggest that aliphatic hydrocarbons are likely to form in preference to their aromatic counterparts. Furthermore, in some catalytic systems it has been noted that aromatic hydrocarbon impurities in the synthesis gas inactivate the synthesis catalyst, and one way speculate that a number of known synthesis catalysts intrinsically are not capable of producing aromatic hydrocarbons.

The wide range of catalysts and catalyst modifications disclosed in the art and an equally wide range of conversion conditions for the reduction of carbon monoxide by hydrogen provide considerable flexibility toward obtaining selected products. A review of the status of this art is given in "Carbon Monoxide-Hydrogen Reactions," Encyclopedia of Chemical Technology, Edited by Kirk-Othmer, 2nd Edition, Volume 4, pp. 446—488, Interscience Publishers, New York, N.Y.

Oxygenated compounds and hydrocarbons are produced in varying proportions in the conversion of synthesis gas. This is understandable if, as proposed by some researchers in the field, the hydrocarbons arise via oxygenated intermediates such as alcohols. By selection of less active catalysts such as zinc oxide, it is possible to obtain oxygenated compounds as the major product. One particular commercial conversion is used to produce methanol from synthesis gas with substantially no coproduction of hydrocarbons. Suitable catalysts are those comprising zinc oxide, in admixture with promoters. Copper or copper oxide may be included in the catalyst composition. Particularly suitable are oxide catalysts of the zinc-copper-alumina type. Compositions of the type described are those currently used in commercial methanol synthesis. Contact of the synthesis gas with the methanol synthesis catalyst is conducted under pressure of 25 to 600 atmospheres ($2.53\times10^3$ to $6.06\times10^4$), preferably 50 to 400 atmospheres ($5.05\times10^3$ to $4.04\times10^4$ kPa), and at a temperature of 400° to 750°F (204° to 399°C). The preferred gas space velocity is within the range of 1,000 to 50,000 volume hourly space velocity measured at standard temperature and pressure. It is noted that the conversion per pass is from about 10% of the carbon monoxide fed to about 30%, i.e. in this process the unconverted synthesis gas must be separated from the methanol product and recycled.

Crystalline zeolites have been contacted with methanol under catalytic conversion conditions. U.S. Pat. No. 3,036,134 shows a 98.4% conversion of methanol to dimethyl ether over sodium X zeolite at 260°C; 1.6 mole% of the product is a mixture of olefins up to pentene, with butene the predominant product. Conversion of methanol over rare earth exchanged and zinc exchanged X zeolite has been reported to produce some hexanes and lighter hydrocarbons (see Advances in Catalysis, Vol. 18, p. 309, Acadamic Press, New York, 1968). It has recently been discovered that alcohols, ethers, carbonyl and their analogous compounds may be converted to higher hydrocarbons, particularly high octane gasoline, by catalytic contact with a special type zeolite catalyst. This conversion is described in U.S. Patents 3,894,106; 3,894,107; 3,907,915.

An object of this invention therefor is to increase the yield of products rich in benzene, toluene, and xylenes (i.e. greater than 50 percent aromatics) when oxygenated hydrocarbons such as methanol and dimethyl ether and product streams containing these and other oxygenated hydrocarbon compounds are converted to benzene, toluene and xylene.

Accordingly, the invention resides in a process for producing a mixture of hydrocarbons comprising:

(a) converting a fossil fuel to a synthesis gas;

(b) converting said synthesis gas to oxygenated hydrocarbons; and

(c) contacting said oxygenated hydrocarbons with a ZSM-5 type zeolite to produce a hydrocarbon stream; characterized by including the further steps of:

(d) contacting at least the hydrocarbon stream from (c) with a catalyst comprising a metal-promoted porous crystalline zeolite having a constraint index within the range of 1 to 12 and a silica to alumina ratio of at least 12 to produce an effluent stream rich in benzene, toluene and xylene; and

(e) removing benzene, toluene and xylene from said effluent stream.

Preferably, the synthesis gas has a volumetric ratio of hydrogen to carbon monoxide plus carbon dioxide of from 1.0 to 6.0.

Preferably the zeolite used in step (d) is of the ZSM-5 type and is impregnated with one or more metals selected from the group consisting of Group VIIIA transition metals, and metals from Group IIB and IIIB. Group VIIIA metals include iron, cobalt, nickel, ruthenium, rhodium, palladium, osmium, iridium and platinum. The Group IIB metals include zinc, cadmium, and mercury. The Group IIIB metals include aluminum, gallium, indium and thallium. Preferred metals are platinum, gallium and zinc, and mixtures of two or more thereof, with gallium being particularly preferred.

The platinum, zinc or gallium in the catalyst composition preferably used in step (d) can be present as the metal oxide and/or as metal ions if cations in the ZSM-5 type zeolite have been exchanged with the metal ions therein. In the case where the cations in the zeolite have been exchanged for metal ions, the metal ion is suitably provided as an aqueous solution of metal salts, such as, for instance, the soluble

nitrate, chloride or sulfate salts of platinum, zinc or gallium. Such catalysts may be produced by conventional ion exchange techniques and the catalysts so produced subsequently dried. For example, an aqueous solution of the metal compound such as tetramine platinum chloride, zinc chloride, or gallium nitrate may be placed in contact with zeolite at ambient or elevated temperature, e.g. by refluxing. The exchanged zeolite is then separated by decantation followed by filtration, washed several times with deionized water and finally dried. Before addition to the aqueous solution of the metal compound, the zeolite may be acid treated.

Alternatively the platinum, zinc, or gallium may only be impregnated on the surface of the zeolite or incorporated in the intra-crystalline zeolite cavities as a metal compound which gives rise to a metal oxide during activation of the catalyst prior to contact with the hydrocarbon feedstock.

It is, however, to be appreciated that where the catalyst composition is prepared by using a metal compound which ionizes in aqueous solution, for example, gallium nitrate, it is inevitable that some of the metal ions will be exchanged with the cations in the zeolite even if the preparation was directed to impregnation of the zeolite.

Whichever method of catalyst preparation is used, the amount of metal present in the catalyst compositions (metal plus zeolite) may suitably vary between 0.01 and 5 percent, preferably between 0.05 and 2 percent by weight. Obviously, a mixture of two or more metals can be incorporated into the zeolite by the methods discussed above. If zinc is present in the catalyst, it is preferred also to include palladium and/or gallium.

The ZSM-5 type crystalline zeolites utilized herein are members of a class of zeolitic materials which exhibit unusual properties. Although these zeolites have unusually low alumina contents, i.e. high silica to alumina mole ratios, they are very active even when the silica to alumina mole ratio exceeds 30. The activity is surprising since catalytic activity is generally attributed to framework aluminum atoms and/or cations associated with these aluminum atoms. These zeolites retain their crystallinity for long periods in spite of the presence of steam at high temperature which induces irreversible collapse of the framework of other zeolites, e.g. of the X and A type. Furthermore, carbonaceous deposits, when formed, may be removed by burning at higher than usual temperatures to restore activity. These zeolites, used as catalysts, generally have low coke-forming activity and therefore are conducive to long times on stream between regenerations by burning carbonaceous deposits with oxygen-containing gas such as air.

An important characteristic of the crystal structure of this class of zeolites is that it provides a selective constrained access to and egress from the intracrystalline free space by virtue of having an effective pore size intermediate between the small pore Linde A and the large pore Linde X, i.e. the pore windows of the structure are of about a size such as would be provided by 10-membered rings of silicon atoms interconnected by oxygen atoms. It is to be understood, of course, that these rings are those formed by the regular disposition of the tetranedra making up the anionic framework of the crystalline zeolite, the oxygen atoms themselves being bonded to the silicon (or aluminum, etc.) atoms at the centers of the tetrahedra.

The silica to alumina mole ratio referred to may be determined by conventional analysis. This ratio is meant to represent, as closely as possible, the ratio in the rigid anionic framework of the zeolite crystal and to exclude aluminum in the binder or in cationic or other form within the channels. Although zeolites with silica to alumina mole ratios of at least 12 are useful, it is preferred in some instances to use zeolites having substantially higher silica/alumina ratios, e.g. 1600 and above. In addition, zeolites as otherwise characterized herein but which are substantially free of aluminum, that is zeolites having silica to alumina mole ratios of up to infinity, are found to be useful and even preferable in some instances. Such "high silica" or "highly siliceous" zeolites are intended to be included within this description. Also included within this definition are substantially pure silica analogs of the useful zeolites described herein, that is to say those zeolites having no measurable amount of aluminum (silica to alumina mole ratio of infinity) but which otherwise embody the characteristics disclosed.

This class of zeolites, after activation, acquire an intracrystalline sorption capacity for normal hexane which is greater than that for water, i.e. they exhibit "hydrophobic" properties. This hydrophobic character can be used to advantage in some applications.

The class of zeolites useful herein have an effective pore size such as to freely sorb normal hexane. In addition, the structure must provide constrained access to larger molecules. It is sometimes possible to judge from a known crystal structure whether such constrained access exists. For example, if the only pore windows in a crystal are formed by 8-membered rings of silicon and aluminum atoms, then access by molecules of larger cross-section than normal hexane is excluded and the zeolite is not of the desired type. Windows of 10-membered rings are preferred, although in some instances excessive puckering of the rings or pore blockage may render these zeolites ineffective.

Although 12-membered rings in theory would not offer sufficient constraint to produce advantageous conversions, it is noted that the puckered 12-ring structure of TMA offretite does show some constrained access. Other 12-ring structures may exist which may be operative for other reasons and, therefore, it is not the present intention to entirely judge the usefulness of a particular zeolite solely from theoretical structural considerations.

Rather than attempt to judge from crystal structure whether or not a zeolite possesses the necessary constrained access to molecules of larger cross-section than normal paraffins, a simple determination of

the "Constraint Index" may be used. The meaning of Constraint Index and its method of determination are fully described in, for example, U.S. Patent No. 3,905,915.

The preferred zeolites used herein are ZSM-5, ZSM-11, ZSM-12, ZSM-23, ZSM-35, ZSM-38, ZSM-48, and other similar materials, ZSM-5 being especially preferred.

ZSM-5 is described in U.S. Patent Nos. 3,702,886 and Re 29,948. ZSM-11 is described in U.S. Patent No. 3,709,979. ZSM-12 is described in U.S. Patent No. 3,832,449. ZSM-23 is described in U.S. Patent No. 4,076,842. ZSM-35 is described in U.S. Patent No. 4,016,245. ZSM-38 is described in U.S. Patent No. 4,046,859. ZSM-48 is described in published European Patent Application No. 15,132.

These specific zeolites described, when prepared in the presence of organic cations, are substantially catalytically inactive, possibly because the intra-crystalline free space is occupied by organic cations from the forming solution. They may be activated by heating in an inert atmosphere at 540°C for one hour, for example, followed by base exchange with ammonium salts followed by calcination at 540°C in air. The presence of organic cations in the forming solution may not be absolutely essential to the formation of this type zeolite; however, the presence of these cations does appear to favor the formation of this class of zeolite. More generally, it is desirable to activate this type catalyst by base exchange with ammonium salts followed by calcination in air at about 540°C for from about 15 minutes to about 24 hours.

Natural zeolites may sometimes be converted to zeolite structures of the class herein identified by various activation procedures and other treatments such as base exchange, steaming, alumina extraction and calcination, alone or in combinations. Natural minerals which may be so treated include ferrierite, brewsterite, stilbite, dachiardite, epistilbite, heulandite, and clinoptilolite.

Preferably, the zeolites have a crystal framework density, in the dry hydrogen form, of not less than about 1.6 grams per cubic centimeter. The dry density for known structures may be calculated from the number of silicon plus aluminum atoms per 1000 cubic Angstroms, as described on page 19 of the article Zeolite Structure by W. M. Meier, included in Proceedings of the Conference in Molecular Sieves, (London, April 1967) published by the Society of Chemical Industry, London, 1968.

When the crystal structure is unknown, the crystal framework density may be determined by classical pyknometer techniques. For example, it may be determined by immersing the dry hydrogen form of the zeolite in an organic solvent which is not sorbed by the crystal. Or, the crystal density may be determined by mercury porosimetry, since mercury will fill the interstices between crystals but will not penetrate the intracrystalline free space.

When synthesized in the alkali metal form, the zeolite is conveniently converted to the hydrogen form, generally by intermediate formation of the ammonium form as a result of ammonium ion exchange and calcination of the ammonium form to yield the hydrogen form. In addition to the hydrogen form, other forms of the zeolite wherein the original alkali metal has been reduced to less than about 1.5 percent by weight may be used. Thus, the original alkali metal of the zeolite may be replaced by ion exchange with other suitable metal cations of Groups I through VIII of the Periodic Table, including, by way of example, nickel, copper, zinc, palladium, calcium or rare earth metals.

In practicing a particularly desired chemical conversion process, it may be useful to incorporate the above-described crystalline zeolite with a matrix comprising another material resistant to the temperature and other conditions employed in the process. Such matrix material is useful as a binder and imparts greater resistance to the catalyst for the severe temperature, pressure and reactant feed stream velocity conditions encountered in many cracking processes.

Useful matrix materials include both synthetic and naturally occurring substances, as well as inorganic materials such as clay, silica and/or metal oxides. The latter may be either naturally occurring or in the form of gelatinous precipitates or gels including mixtures of silica and metal oxides. Naturally occurring clays which can be composited with the zeolite include those of the montmorillonite and kaolin families, which families include the sub-bentonites and the kaolins commonly known as Dixie, McNamee-Georgia and Florida clays or others in which the main mineral constituent is halloysite, kaolinite, dickite, nacrite or anauxite. Such clays can be used in the raw state as originally mined or initially subjected to calcination, acid treatment or chemical modification.

In addition to the foregoing materials, the zeolites employed herein may be composited with a porous matrix material, such as alumina, silica-alumina, silica-magnesia, silica-zirconia, silica-thoria, silica-beryllia, and silica-titania, as well as ternary compositions, such as silica-alumina-thoria, silica-alumina-zirconia, silica-alumina-magnesia and silica-magnesia-zirconia. The matrix may be in the form of a cogel. The relative proportions of zeolite component and inorganic oxide gel matrix, on an anhydrous basis, may vary widely with the zeolite content ranging from between 1 to 99 percent by weight and more usually in the range of 5 to 80 percent by weight of the dry composite.

In the process of the invention, the oxygenated hydrocarbon stream obtained by conversion of synthesis gas is contacted with ZSM-5 and then the resultant hydrocarbon stream is contacted with a catalyst comprising a zeolite as described above, preferably ZSM-5, and a Group VIII metal, preferably platinum, a Group IIB metal, preferably zinc, and/or a Group IIIB metal, preferably gallium.

The accompanying drawing is a flow diagram of a process according to one example of the invention.

Referring to the drawing, coal, shale oil, or residua, or a combination thereof, is conveyed via line 1, 2 or 3, respectively and thence via line 4 to a synthesis gas plant, 5, where it is converted to synthesis gas. If hydrogen sulfide is produced in this plant, it may be separated and sent via line 6 to a treatment plant (not

shown) for sulfur recovery. Synthesis gas, previously treated in a catalytic carbon monoxide shift converter and then reduced in carbon dioxide content by selective sorption, is then conveyed via line 7 to a first reaction zone 8, where it is at least partially catalytically converted to produce a carbon monoxide reduction product that contains at least 20% by weight of oxygenated products, preferably methanol and/or dimethyl ether. Part or all of the unconverted synthesis gas may be separated from such reduction product and recycled via line 10.

That portion of reaction product not recycled through line 10 is conveyed to the second reaction zone 11 where catalytic conversion to hydrocarbons and steam occurs. The reaction products from the second reaction zone 11 can then be diverted through line 27, open valve 31 to the reactor 33 where it is contacted with the ZSM-5 type metal impregnated porous crystalline zeolite.

The conversion in the second reaction zone 11 is conducted at a temperature of 250 to 650°C, preferably 300 to 650°C, a pressure of subatmospheric 1 to 50 atmospheres (100—5,000 kPa) and at a liquid hourly space velocity.

In an alternative the reaction products in line 12 are conveyed to a cooler, 13, and the cooled products are then conveyed via line 14 to a separator 15; note that the cooler 13, line 14 and separator 15 may be one integral unit. Water is removed from the separator 15 via line 16, gas is removed via line 17, and liquid hydrocarbon product via line 18. The liquid hydrocarbon product is conveyed via line 18 to reactor 33 where it is contacted with the metal impregnated crystalline zeolite preferably ZSM-5.

Effluent from the reactor 33 is conveyed through to a cooler and separator (not shown) and the liquid portion thereof is fractionated in a distillation tower 19 into fractions consisting of benzene, toluene and xylene. Any fraction containing hydrocarbons in excess of 9 carbon atoms can be recycled via line 35 through reactor 33 for conversion to benzene, toluene and xylene.

Additional details of the operating conditions in carbon oxide converter 8 and converter 11 are set forth in U.S. Patent 4,076,761.

As indicated previously the reactor 33 contains ZSM-5 type zeolite, preferably in the form of a fixed bed. Preferably the ZSM-5 zeolite is in the hydrogen or acid form and has deposited thereon the equivalent in metal of between .01 and 1 percent of gallium of the total composition. In a typical, and preferred embodiment, the feedstream of oxygenated hydrocarbons is introduced into the reaction zone 33 at a temperature within the range of 350°C to 650°C, a pressure within the range of $1.\times10^5$ to $30\times10^5$ pascal (0 to 400 psig), and a WHSV of 0.1 to 10.

Preferred temperatures in the reaction zone 33 fall within the range of 400°C to 600°C and preferred pressures fall within the range of $1.\times10^5$ to $15\times10^5$ pascal (0 to 200 psig). A preferred WHSV is between 0.2 and 3.

Example 1

Samples of acid or hydrogen-form ZSM-5 zeolite catalyst prepared in the form of extrudate were impregnated respectively with gallium, zinc, palladium, and platinum in the concentrations shown in Table 1. A hydrocarbon feedstock obtained from the treatment of methanol through a dehydration reactor and then further passed over a ZSM-5 catalyst zone corresponding to reaction zone 11 was then flowed over each catalyst under the conditions shown in Table 1. The results obtained show a substantial increase in the production of aromatics, namely benzenes, toluene and xylene,

In Tables II—V are shown the results obtained with the same feedstock and catalysts at different conditions of temperature and pressure. In Tables I—V the composition of the feedstock is shown in the column headed "Feed." For the sake of convenience, in the Tables the abbreviations BTX and BZ are used respectively to mean benzene, toluene, xylene and benzene.

TABLE I

| Example No. | Feed | 1 | 2 | 3 | 4 | 5 |
|---|---|---|---|---|---|---|
| Catalyst | | HZSM-5 | Ga/HZSM-5 | Zn/Pd HZSM-5* | Pt/HZSM-5 | Ga/HZSM-5 |
| Metal concentration, wt. % | | 0 | 0.5 | 2/1 | 0.1 | 0.5 |
| Temp., °C (°F) | | | | 537.8 (1000) | | |
| Pressure kPa, (psig) | | | | 101.4 (0) | | |
| WHSV | | | | 1 | | 0.5 |
| Conc. benzene, toluene and xylene in product, wt % | 14.3 | 49.5 | 60.3 | 63.8 | 51.7 | 77.5 |
| Conc. of $C_9^+$ in product, wt % | 26.4 | 15.8 | 7.5 | 8.8 | 11.0 | 10.3 |
| $C_9^+$ converted, wt % | | 40.1 | 71.6 | 66.7 | 58.3 | 61.0 |

*40/1 mole ratio of $SiO_2$ to $Al_2O_3$ HZSM-5, all others 70/1 $SiO_2/Al_2O_3$

TABLE II

| Run No. | Feed | 1-1 | 1-2 | 1-3 |
|---|---|---|---|---|
| Catalyst | | —HZSM-5*— | | |
| Temperature, (°F) °C | | (1000) 537.8 | (1050) 565.6 | (1100) 593.3 |
| Pressure: 101.4 kPa, (0 psig) | | | | |
| WHSV: 1 | | | | |
| Conc. of BTX in prod. wt. % | 14.3 | 49.5 | 55.6 | 58.7 |
| Prod. distribution, wt % | | | | |
| $C_1$—$C_3$ | 1.3 | 32.8 | 30.5 | 21.8 |
| $C_4$—$C_9$ (non-aromatics) | 58.0 | 1.7 | 0.5 | 0.2 |
| BZ | 0.2 | 11.0 | 16.5 | 18.1 |
| Toluene | 2.7 | 23.0 | 25.7 | 26.9 |
| Xylenes | 11.4 | 15.5 | 13.4 | 13.7 |
| $C_9^+$ aromatics | 19.6 | 9.6 | 9.0 | 7.8 |
| Durenes | 6.8 | 6.2 | 4.4 | 11.5 |
| Total | 100.0 | 100.0 | 100.0 | 100.0 |

*70/1 $SiO_2/Al_2O_3$ HZSM-5, no added metal.

TABLE III

| Run No. | Feed | 2-1 | 2-2 | 2-3 | 2-4 | 2-5 |
|---|---|---|---|---|---|---|
| Catalyst | | ——— Ga/HZSM-5* ——— | | | | |
| Temperature, °C | | 537.8 | 532.8 | 565.6 | 510 | 510 |
| Temperature, °F | | 1000 | 1000 | 1050 | 950 | 950 |
| Pressure: 101.4 kPa (0 psig) | | | | | | |
| WHSV | | 1 | 0.5 | 1 | 1 | 0.5 |
| Conc. of BTX in prod. wt % | 14.3 | 60.3 | 77.5 | 59.6 | 51.2 | 72.7 |
| Product distribution | | | | | | |
| $C_1$—$C_3$ | 1.3 | 31.0 | 11.7 | 23.7 | 32.8 | 16.2 |
| $C_4$—$C_9$ (non-aromatics) | 58.0 | 0.4 | 0.1 | 0.3 | 2.9 | 0.9 |
| BZ | 0.2 | 15.7 | 15.6 | 17.6 | 11.4 | 12.6 |
| Toluene | 2.7 | 28.9 | 39.6 | 27.5 | 24.6 | 36.2 |
| Xylenes | 11.4 | 15.7 | 21.7 | 14.5 | 15.2 | 23.9 |
| $C_9^+$ aromatics | 19.6 | 5.8 | 5.4 | 11.2 | 7.8 | 7.2 |
| Durenes | 6.8 | 1.7 | 4.9 | 5.1 | 5.1 | 3.0 |
| Total | 100.0 | 99.2 | 99.6 | 100.0 | 100.0 | 100.0 |

*70/1 $SiO_2/Al_2O_3$ HZSM-5, containing 0.5 weight percent of gallium

TABLE IV

| Run No. | Feed | 3-1 | 3-2 | 3-3 | 3-4 |
|---|---|---|---|---|---|
| Catalyst | | Zn/Pd/HZSM-5* | | Zn/Pd/HZSM-5** | |
| Temperature, °C | | 371.1 | 426.7 | 426.7 | 537.8 |
| Temperature, °F | | 700 | 800 | 800 | 1000 |
| Pressure, 101.4 kPa, (0 psig) | | | | | |
| WHSV | | 1 | 1 | 3 | 1 |
| Conc. of BTX in prod. wt. % | 14.3 | 37.6 | 35.9 | 39.8 | 63.8 |
| Prod. distribution wt. % | | | | | |
| $C_1$—$C_3$ | 1.3 | 13.3 | 26.1 | 18.7 | 27.2 |
| $C_4$—$C_9$ (non-aromatics) | 58.0 | 26.1 | 14.2 | 19.0 | 0.1 |
| BZ | 0.2 | 2.7 | 3.6 | 3.0 | 18.8 |
| Toluene | 2.7 | 14.5 | 12.9 | 15.5 | 30.5 |
| Xylenes | 11.4 | 20.6 | 19.4 | 21.3 | 14.5 |
| $C_9^+$ aromatics | 19.6 | 14.6 | 15.1 | 12.4 | 5.9 |
| Durenes | 6.8 | 8.2 | 8.7 | 10.2 | 2.9 |
| Total | 100.0 | 100.0 | 100.0 | 100.1 | 99.9 |

*70/1 $SiO_2/Al_2O_3$ HZSM-5 containing 2 percent by weight of zinc and 1% by weight of palladium.
**40/1 $SiO_2/Al_2O_3$ HZSM-5 containing 2 percent by weight of zinc and 1% by weight of palladium.

TABLE V

| Run No. | Feed | 4-1 | 4-2 | 4-3 | 4-4 | 4-4 |
|---|---|---|---|---|---|---|
| Catalyst | | Pt/HZSM-5* | | | | |
| Temperature, °C | | 371.1 | 426.7 | 482.2 | 537.8 | 565.6 |
| Temperature, °F | | 700 | 800 | 900 | 1000 | 1050 |
| Pressure, 101.4 kPa (0 psig) | | | | | | |
| WHSV: 1 | | | | | | |
| Conc. of BTX in prod. wt. % | 14.3 | 36.9 | 29.6 | 39.4 | 51.7 | 56.2 |
| Prod. distribution wt.% $C_1$—$C_3$ | 1.3 | 18.3 | 41.5 | 36.0 | 36.7 | 33.0 |
| $C_4$—$C_9$ (non-aromatics) | 58.0 | 30.2 | 22.6 | 7.4 | 0.5 | 0.2 |
| BZ | 0.2 | 2.6 | 5.0 | 6.3 | 13.1 | 17.9 |
| Toluene | 2.7 | 13.3 | 13.7 | 17.1 | 24.4 | 26.7 |
| Xylenes | 11.4 | 21.0 | 10.9 | 16.0 | 14.2 | 11.6 |
| $C_9^+$ aromatics | 19.6 | 11.1 | 5.3 | 9.9 | 7.0 | 8.0 |
| Durenes | 6.8 | 4.0 | 1.0 | 7.4 | 4.0 | 2.5 |
| Total | 100.0 | 100.0 | 100.0 | 100.0 | 100.0 | 100.0 |

*70/1 $SiO_2/Al_2O_3$ HZSM-5, containing 0.1 percent by weight of platinum.

**Claims**

1. A process for producing a mixture of hydrocarbons comprising:
(a) converting a fossil fuel to a synthesis gas;
(b) converting said synthesis gas to oxygenated hydrocarbons; and
(c) contacting said oxygenated hydrocarbons with a ZSM-5 type zeolite to produce a hydrocarbon stream; characterized by including the further steps of:
(d) contacting at least the hydrocarbon stream from (c) with a catalyst comprising a metal-promoted porous crystalline zeolite having a constraint index within the range of 1 to 12 and a silica to alumina ratio of at least 12 to produce an effluent stream rich in benzene, toluene and xylene; and
(e) removing benzene, toluene and xylene from said effluent stream.

2. The process of Claim 1 wherein the metal of said catalyst composition of (d) comprises zinc, cadmium, mercury, aluminum, gallium, indium, thallium, iron, cobalt, nickel, ruthenium, rhodium, palladium, osmium, indium, platinum or a mixture of two or more of these.

3. The process of Claim 1 or Claim 2 wherein said ZSM-5 type zeolite of (c) is a catalytically active porous crystalline zeolite having a silica to alumina ratio of at least 12, a constraint index of 1 to 12 and a crystal density, in the hydrogen form, of not substantially below 1.6 grams per cubic centimeter, and said contacting is conducted at a temperature of 250° to 650°C, a space velocity of 0.1 to 50 liquid hourly space velocity, and a pressure of 1 to 50 atmospheres (100—5000 kPa).

4. The process of any preceding Claim wherein the hydrocarbon stream from (c) is cooled and the non-aqueous liquid fraction thereof is contacted with said catalyst of step (d).

5. The process of any preceding Claim wherein the conditions of (d) include a temperature of from 300 to 650°C, a pressure of from $1 \times 10^5$ to $30. \times 10^5$ pascal and a WHSV of from 0.1 to about 10.

6. The process of any preceding Claim wherein said zeolite of (d) is selected from the group consisting of ZSM-5, ZSM-11, ZSM-12, ZSM-23, ZSM-35, ZSM-38, and ZSM-48.

7. The process of Claim 6 wherein said zeolite of (d) is ZSM-5.

8. The process of any preceding Claim wherein the catalyst of (d) contains between 0.01 and 5 percent of metal by weight of the overall catalyst.

9. The process of any preceding Claim wherein the metal in the catalyst of (d) is gallium.

10. The process of any one of Claims 1 to 8 wherein the metal of the catalyst of (d) is zinc together with palladium and/or gallium.

**Patentansprüche**

1. Verfahren zur Herstellung einer Kohlenwasserstoffmischung, welches umfaßt:

(a) Umwandlung eines fossilen Brennstoffes zu Synthesegas;

(b) Umwandlung des Synthesegases zu mit Sauerstoff angereicherten Kohlenwasserstoffen;

(c) Inkontaktbrigen der mit Sauerstoff angereicherten Kohlenwasserstoffe mit einem Zeolith vom ZSM-5-Typ, um einen Kohlenwasserstoffstrom herzustellen; dadurch gekennzeichnet, daß die weiteren Stufen eingeschlossen sind:

(d) Inkontaktbringen zumindest des Kohlenwasserstoffstromes aus (c) mit einem Katalysator, der einen mit Metall aktivierten porösen kristallinen Zeolith mit einem Zwangsindex im Bereich von 1 bis 12 und einem Siliziumdioxid/Aluminiumoxyd-Verhältnis von mindestens 12 umfaßt, um einen Abflußstrom zu erzeugen, der reich an Benzol, Toluol und Xylol ist; und

(e) Entfernen von Benzol, Toluol und Xylol aus dem Abflußstrom.

2. Verfahren nach Anspruch 1, worin das Metall der Katalysatorzusammensetzung von (d) Zink, Kadmium, Blei, Aluminium, Gallium, Indium, Thallium, Eisen, Kobalt, Nickel, Ruthenium, Rhodium, Palladium, Osmium, Indium, Platin oder eine Mischung von zwei oder mehreren davon umfaßt.

3. Verfahren nach Anspruch 1 oder 2, worin der Zeolith vom ZSM-5-Typ von (c) ein katalytisch aktiver poröser kristalliner Zeolith mit einem Siliziumdioxyd/Aluminiumoxyd-Verhältnis von mindestens 12, einem Zwangsindex von 1 bis 12 und einer Kristalldichte in der Wasserstofform von nicht wesentlich weniger als 1,6 g/cm$^3$ ist und das Inkontaktbringen bei einer Temperatur von 250 bis 650°C, einer Raumgeschwindigkeit von 0,1 bis 50 stündliche Flüssigkeit-Raum-Geschwindigkeit und einem Druck von 1 bis 50 Atmosphären (100 bis 5000 kPa) durchgeführt wird.

4. Verfahren nach einem der vorstehenden Ansprüche, worin der Kohlenwasserstoffstrom von (c) abgekühlt wird, und die nicht-wässrige flüssige Fraktion davon mit dem Katalysator der Stufe (d) inkontaktgebracht wird.

5. Verfahren nach einem der vorstehenden Ansprüche, worin die Bedingungen von (d) eine Temperatur von 300 bis 650°C, einen Druck von $1\times10^5$ bis $30\times10^5$ Pa und eine WHSV von 0,1 bis etwa 10 umfassen.

6. Verfahren nach einem der vorstehenden Ansprüche, worin der Zeolith von (d) aus der Gruppe ausgewählt ist, die aus ZSM-5, ZSM-11, ZSM-12, ZSM-23, ZSM-35, ZSM-38 und ZSM-48 besteht.

7. Verfahren nach Anspruch 6, worin der Zeolith von (d) ZSM-5 ist.

8. Verfahren nach einem der vorstehenden Ansprüche, worin der Katalysator von (d) zwischen 0,01 und 5% an Metall bezogen auf das Gewicht des gesamten Katalysators enthält.

9. Verfahren nach einem der vorstehenden Ansprüche, worin das Metall in dem Katalysator von (d) Gallium ist.

10. Verfahren nach einem der Ansprüche 1 bis 8, worin das Metall des Katalysators von (d) Zink zusammen mit Palladium und/oder Gallium ist.

**Revendications**

1. Procédé de préparation d'un mélange d'hydrocarbures comprenant:

(a) la conversion d'un fuel fossile en un gaz de synthèse;

(b) la conversion dudit gaz de synthèse en hydrocarbures oxygénés; et

(c) la mise au contact desdits hydrocarbures oxygénés avec une zéolite du type ZSM-5 pour produire un courant d'hydrocarbures; caractérisé par l'introduction des étapes ultérieures suivantes:

(d) la mise au contact au moins du courant d'hydrocarbures en provenance de l'étape (c) avec un catalyseur comprenant une zéolite cristalline poreuse contenant un promoteur métallique, ayant un indice de contrainte compris entre 1 et 12 et un rapport silice/alumine d'au moins 12, pour produire un effluent riche en benzène, toluène et xylène; et

(e) la séparation du benzène, du toluène et du xylène dudit effluent.

2. Procédé selon la revendication 1, dans lequel le métal de la composition catalytique de (d) comprend du zinc, du cadmium, du mercure, de l'aluminium, du gallium, de l'indium, du thallium, du fer, du cobalt, du nickel, du ruthénium, du rhodium, du palladium, de l'osmium, de l'indium, du platine ou un mélange de deux ou davantage du ces métaux.

3. Procédé selon la revendication 1 ou 2, dans lequel la zéolite de type ZSM-5 de l'étape (c) est une zéolite cristalline poreuse, active catalytiquement, ayant un rapport silice/alumine d'au moins 12, un indice de contrainte de 1 à 12 et une densité de cristal sous la forme hydrogénée peu inférieure à 1,6 g/cm$^3$, et ladite mise au contact est effectuée à une température comprise entre 250 et 640°C, avec une vitesse spatiale de 0,1 à 50 (vitesse spatiale horaire liquide) et à une pression de 1 à 50 atmosphères (100 à 5 000 kPa).

4. Procédé selon l'une quelconque des revendications précédentes, dans lequel le courant

d'hydrocarbures en provenant de l'étape (c) est refroidi et la fraction liquide non aqueuse est mise au contact dudit catalyseur de l'étape (d).

5. Procédé selon l'une quelconque des revendications précédentes, dans lequel les conditions de l'étape (d) comprennent une température comprise entre 300 et 650°C, une pression comprise entre $1\times10^5$ et $30\times10^5$ Pascal et une VSHP comprise entre 0,1 et 10 environ.

6. Procédé selon l'une quelconque des revendications précédentes, dans lequel ladite zéolite de l'étape (d) est choisie dans le groupe comprenant les ZSM-5, ZSM-11, ZSM-12, ZSM-23, ZSM-35, ZSM-38, ZSM-48.

7. Procédé selon la revendication 6, dans lequel ladite zéolite de l'étape (d) est la ZSM-5.

8. Procédé selon l'une quelconque des revendications précédentes, dans lequel le catalyseur de l'étape (d) contient entre 0,01 et 5% en poids de métal du catalyseur total.

9. Procédé selon l'une quelconque des revendications précédentes, dans lequel le métal dans le catalyseur de l'étape (d) est le gallium.

10. Procédé selon l'une quelconque des revendications 1 à 8, dans lequel le métal du catalyseur de l'étape (d) est le zinc avec du palladium et/ou du gallium.

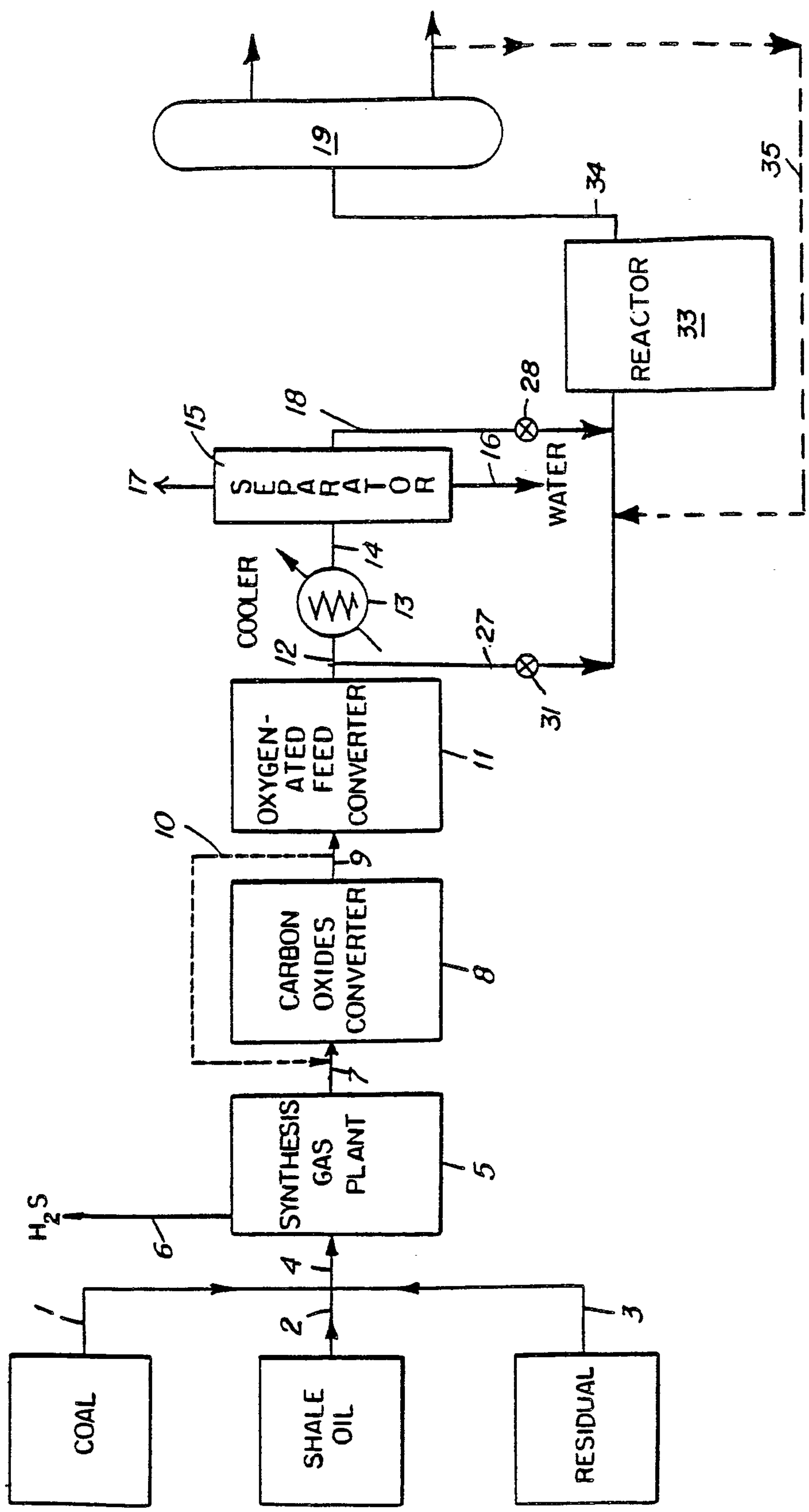
COAL
SHALE OIL
RESIDUAL
1
2
3
4
H2S
6
SYNTHESIS GAS PLANT
5
7
CARBON OXIDES CONVERTER
8
9
10
OXYGEN-ATED FEED CONVERTER
11
12
COOLER
13
14
SEPARATOR
15
16
17
18
WATER
27
31
28
REACTOR
33
34
19
35